# EUROPEAN PATENT APPLICATION

(11) **EP 3 131 030 A1**
(43) Date of publication of application: **15.02.2017**
(21) Application number: 16382209.1
(22) Date of filing: 10.05.2016
(51) Int. Cl.: G06F 19/00

(54) **CENTER FOR MEDICAL ARTIFICIAL INTELLIGENCE CONTROL WITH REMOTE SYSTEM FOR PREPARATION OF DIAGNOSIS, DRUG PRESCRIPTION AND ONLINE MEDICAL TREATMENT SHIPPING VIA TELEMEDICINE**

(30) Priority: 10.08.2015 BR 102015019130
(71) Applicant: Leonardo Pereira, Luis Henrique, 88330-215 Balneario Camboriu, Sao Paulo (BR)
(72) Inventor: Leonardo Pereira, Luis Henrique, 88330-215 Balneario Camboriu, Sao Paulo (BR)
(74) Representative: Urizar Anasagasti, Jesus Maria

(57) **Abstract**

Control center of medical artificial intelligence with remote system for diagnosis preparation, drug prescription and online medical treatment shipping via telemedicine. Developed for medical treatment at a distance, using Medical Artificial Intelligence (MAI) and Telemedicine. The patient (P), using her/his computer, tablet, laptop or through SMS technology using her/his mobile phone, send health information to a control center (C) hosted in the web and in an integrated computer with dedicated software (S) developed with algorithms and filters based in medical artificial intelligence. The software (S) form a database (BD) personalized in clinical analysis, diagnosis, drugs prescription and the proposed treatment to be administered to said patient (P). The detailed history by software (S) in the database (DB) is stored in the control center (C), indicating physicians (MC) also registered according to the specialty to the patient (P), defined by the clinical results obtained. The physician (P) accesses then, from her/his computer, the database (BD) and being with the results of the patient (P) displayed on the screen, check them, validating the attendance and thus beginning to monitor remotely in a custom and legalized manner (under supervision of the control central (C)), requesting and sending information, medical tests, prescriptions and administering prescription drugs in encrypted procedure accessed only by implanted passwords with translation intra software and full mobility, whether via mobile or desktop, independent of the region of the planet. Through the system, physician (P) treatment is speeded on a large scale and in a personalized way to the patient (P), whether in public or private networks.

## Description

Center for medical artificial intelligence control with remote system for preparation of diagnosis, drug prescription and online medical treatment shipping via telemedicine.

The present descriptive report refers to a patent application for an innovative medical care system. In this system, through a Control Center via Medical Artificial Intelligence, the medical professional use the world wide web (internet), being able to treat patients and with no quantity limits, on a worldwide scale regardless of location, language or distance in which they find themselves, through telemedicine. The work is done without barriers in relation to treatment specialty in different languages between physicians and patients and unlimited number of appointments and treatments arising.

The system provides that the registered patient feed a database about their health problems and is instantly diagnosed, getting prescription medications to their problem along with expert guidance and requests for medical tests. The Control Center also elaborates the type of treatment to be performed, from the analysis of health data and properly stored illness from a patient database. This database is analyzed by the center through medical artificial intelligence software. The analysis resulting from the processing are personalized and individualized for each type of problem and patient, taking into account all the clinical data provided when the patient is registered in the system. Then through a Center processor, it is created a custom history for future monitoring of the patient, which is thoroughly studied by the program based on medical parameters contained in its database. Across the center, when accessing the system, the physician, also properly registered and remotely acting, validates the analysis of this preliminary online service based on the data stored by the system. Evaluates and oversees the entire process of prescription, test applications and [003] diagnosis besides monitoring treatment via WEB.

### STATE OF ART

As is of common knowledge, traditional medical treatment is based on personal and on-site relationships between patients and physicians. It is exercised in offices, clinics and hospitals, held in person, which happen when the patient previously schedule date and time, generally through telephone contact, then dislocating to the office for receipt of medical treatment. Among the main problems regarding the presence medicine, it can be mentioned the time spent on scheduling, delays, returns, locomotion, request for medical examinations, situations that worsen when it is necessary repetitive procedures, especially for patients with stable chronic diseases that need the same remedies periodically, successive revenue and examinations, often needed for years.

Another issue, of personal nature, concerns the inevitable exposure of patients in environments such as offices, clinics and hospitals when they are victimized by intimate diseases and sexual health (such as infertility, premature ejaculation, erectile dysfunction, prostate ills, etc.), bringing them discomfort and depression. Added to these problems, traffic increasing in large cities, hampering mobility.

The limited mobility is closely linked to the fact that medical specialists are concentrated physically in specific regions, as local physician polo (which forces the patient to the inevitable displacement, sometimes over great distances).

Nowadays, however, with the advent of computers, the internet and hospital management software, new digital markets have emerged and, among them, the already known telemedicine, i.e. the practice of medicine at a distance. In this medical model occur interventions, diagnostic and treatment decisions based on information transmitted through telecommunication systems, without direct physical contact between physician and patient. These markets of "digital health" called Ehealth and MHealth relate, with great potential, the medical art with technology. Usually, the patient must be registered in a health system database that is used for communication, by their computer, tablet, mobile phone or other. After access. The patient can schedule appointments online through the usual computer programs.

In this sense can be cited the document PI 06036023, called "TELEMEDICINE SYSTEM FOR REMOTE MONITORING OF PATIENTS", filed on 08.25.2006, by which patients are directed to a place of treatment communicating with a monitoring center containing the servers and a database. Through monitoring center, physicians can access the database and can perform a type of service or give medical information to patients.

In other known systems the patient has available at his residence a portable telemedicine unit relating to various medical equipment. This telemedicine unit is connected to a communication network (Internet) for access to a physician that, before analysis, guides the patient relative to medication schedules, dosages, etc.

Therefore, through the use of this information and telecommunications technology the patient receive medical attention, with remedies prescription and actions that can be done remotely. To have an idea of the trend of medical treatment at a distance, even the "Google" will provide health information, although widespread, thus transcribed: *"From the next days, when asking Google about common health conditions, the related medical facts appear to the right of the screen, on the Knowledge Graph. We will show the typical symptoms and treatments as well as details on how it is presented the normal or critical frames, if it is something contagious and affects all ages and much more" (Wall Street Journal)".*

Anyway, the telemedicine system traditionally known collides with the slowness of the unit service and without the support of medical artificial intelligence technologies, still depending on the interaction of the physician in the clinic (usually stuck with busy schedules) with the patient. The internet tends thus to have its agility underutilized. This is accentuated in the case of public health, as a large number of accesses for appointments schedule becomes practically impossible the service on a large scale. Thus, the telemedicine model known is restricted to medical treatment for a patient at a time, without the support of artificial intelligence and even practiced solely in accordance with the requested service, and limited to the same language of the physician/patient.

### Even using management software and online appointments the

Impediments cited in relation to telemedicine end up approaching it to the conventional physician office (in person). Thus, it voids the possible agility afforded by the internet. This speed would be welcome in private or public health, since both continue to rely on a physician who treats without the aid of medical artificial intelligence systems and always in the same languages. As an example of this barrier, the attendance made between different countries requires both patient and physician have knowledge of the language to be spoken that prevents treatments by physician and patients from different countries in different languages.

However, there are currently instruments that can become increasingly viable the service for telemedicine, among which the called Medical Artificial Intelligence (MAI), due to a computer branch by which its technology captures and organizes patients health/disease data, suggesting diagnoses, performing medical, surgical or robotic treatment.

### OBJECTIVE OF PATENT

The system in question, reason of this patent application is precisely to combine the artificial medical intelligence to telemedicine technologies, updating in a globalized world and with less and less boundaries.

Through the proposed system is composed a database with millions of clinical information provided by registered patients, given that these are compiled and analyzed instantly by MAI software (Medical Artificial Intelligence). They are then stored in a control center hosted on the web or computer, which analyzes and evaluates (following medical criteria) in personalized and instantly manner each of these patients to develop diagnostics, predict risk situations and submit proposals for treatment.

The physician in charge, also registered, is indicated by the system according to required specialty access this control center database also via the Internet and has at its disposal the results processed by the software to validate the attendance and assume, legally, the patient treatment. O

physician has full autonomy to change any suggestion given by the Control Center software. The procedure is encrypted preserving the physician/patient confidentiality. Through the speed and agility since the registration, analysis, diagnostics and procedures designed for the treatment, the system becomes perfectly feasible for medicine to the public network, even worldwide.

The other advantages and technical effects of the system in question are described in further detail below, using the attached scheme:

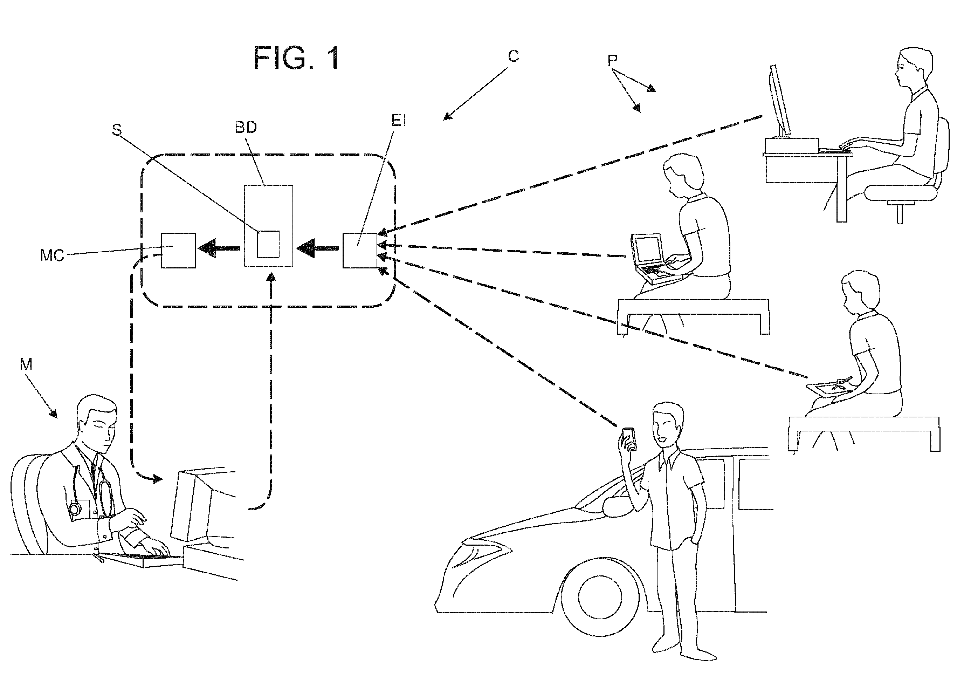

Figure 1 - shows by dashed arrows schematically the future patient accessing the control center for filling out the form related to their health. As noted, the access by the patient (including the physician itself) is highly mobile, from home or in public places, outdoors, finally, at any time, whether by computer, notebook, tablet, including the mobile device (i.e. by mobile or desktop means). The user enters the information in his/her language. Such information is processed by dedicated software of the control center and the clinical results stored in a database that can be accessed by the physician, also registered to the system, which validates the attendance to the beginning of the medical supervision of the patient;

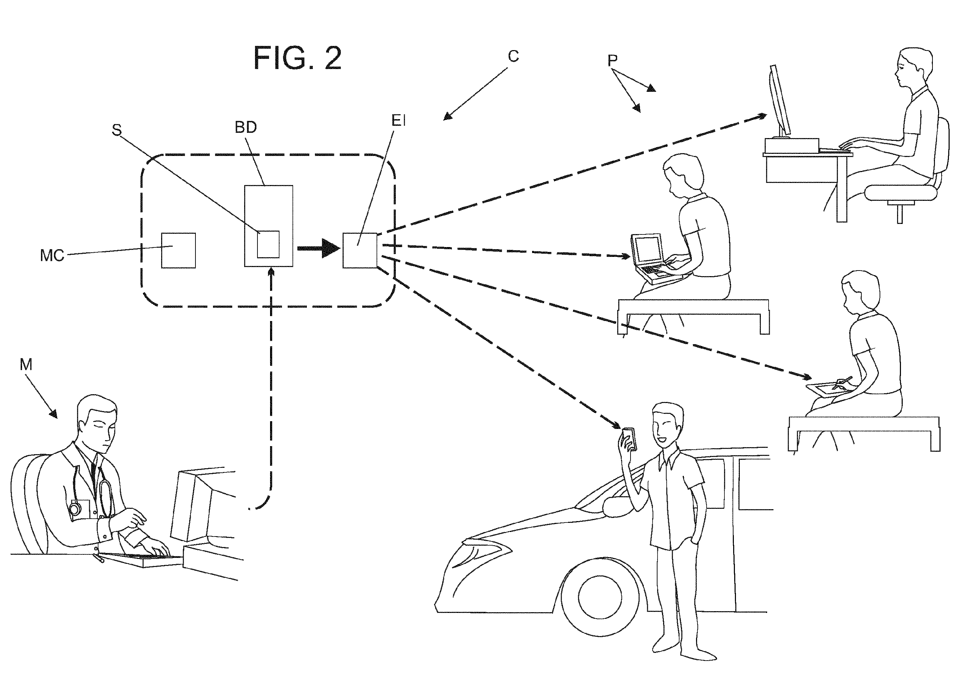

Figure 2 - view according to the previous figure diagram showing by the dashed arrows, that the physician has started monitoring, under the supervision of control center, accessing the patient in simultaneous translation according to the language to be spoken both by mobile means or desktop;

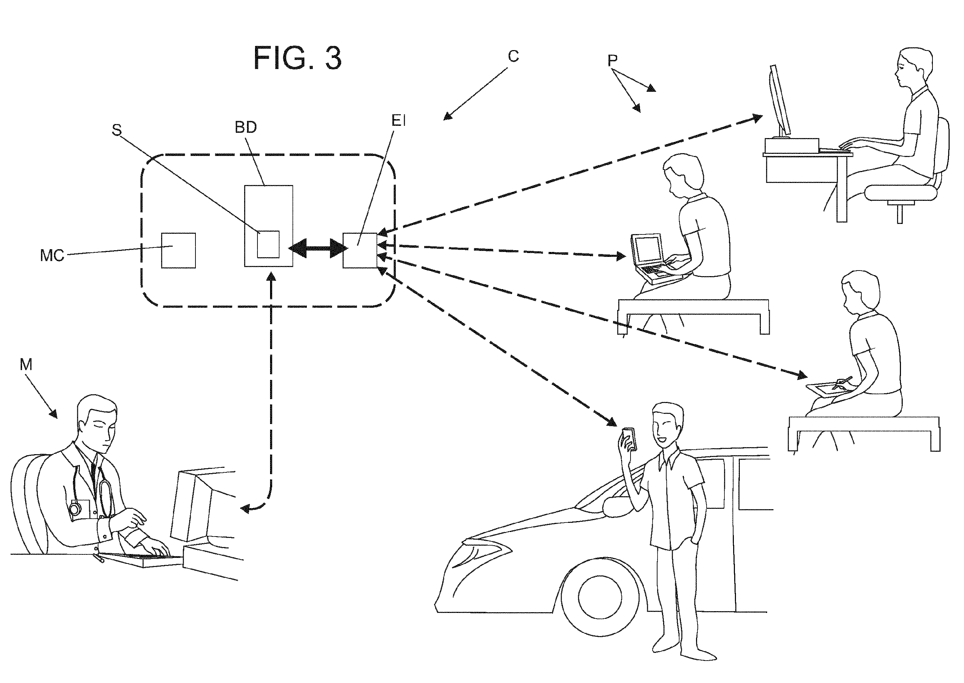

Figure 3 - view according to the previous figure diagram showing by the dashed arrows, the physician/patient interaction communicating in the same language, anywhere and at any time. Thus, all the processing from start to finish is made in the appropriate language thanks to a simultaneous translation system also integrated into the control center, which provides treatment at any time, place and language, worldwide. Therefore, the physician will be guided by the results of the system and, according to her/his understanding, she/he will change or not them during the course of treatment, but working always under the supervision of control center.

In accordance with the Annex scheme, the "Control center for medical artificial intelligence with remote system for diagnosis preparation, drug prescription and online medical treatment shipping via telemedicine" purpose of this invention patent is constituted from a control center (C) in setting up a server hosted on the WEB, operating via internet, which includes an information input (II) communicating with dedicated software (S ), specially developed with medical algorithms and filters based on [023] medical artificial intelligence. The control center (C) also includes a portal with registered physicians (RP) of any regions, states or countries on the planet.

Thus constituted, the control center (C), using the dedicated software (S) will enable an unprecedented health treatment system, combining telemedicine and medical artificial intelligence MAI.

As shown in the drawing in Figure 1, according to the project proposed the patient (P), from anywhere in the world, either via e-mail through her/his computer, tablet, laptop, or through SMS technology using her/his mobile phone, access the information input (II) of the control center (C), virtually filling out a form about her/his health status in native language. Then the dedicated software (S) perform instantly and in detail clinical analysis with diagnosis, remedies prescription as well as the proposed treatment to be administered. The results of this cynical analysis of the patient (P), taking into account their own data on the moment of register are stored in a personalized and individualized database (BD), forming the detailed history by software (S).

Then, the control center (C) accesses its portal of registered physicians (RP) and indicates medical professional (P) whose specialty is defined by the clinical results obtained. The physician (P) then accesses the database (BD) from computer that, as such, has the content of the patient (P) results simultaneously translated into her/his own language, and displayed on the screen, as shown in Figure 1. Thus, the physician check them, validating the attendance and beginning to monitor remotely and in a personalized way. During treatment, the physician (P), with Control Center (C) support, requests and sends medical informative, prescriptions, medical tests (also transported via internet) and give remedies prescription, accompanying the patient (P), as shown in figure 2.

The transmission of such data both by the patient (P) and the physician (P), in short, all the treatment is done with simultaneous translations by specific software (with simultaneous translator program), always adapting to the language to be used, securely, encrypted, accessible only by implanted passwords and in procedure monitored by the control center (C), thus giving full mobility to the patient (P) whether via mobile or desktop, as shown in figure 3.

As noted, through immediate access to the database (BD) by the physician (P) it is made possible personalized service, in quick and plural form, that is, it is allowed the attendance and treatment of a large number of patients (P) by physician (P), in large scale and in real time, in particular solving the problem in public networks with the online medical support from any area of the planet regardless of the spoken language since translations are made intra software. With this "virtual mobility" is eliminated the patient (P) displacement, avoiding all such difficulties as loss of time, embarrassments, wear and financial costs. Thanks to ease of access to clinical results processed by dedicated software (S), it is enabled rapid classification by the physician (P) of patients (P) with risk of cancer, cardiovascular disease, diabetes, etc., speeding procedures and providing the treatment either in the public or private network. Depending on the severity of the case, the system gives greater speed to the request by the physician (P), to presence medical attention.

## Claims

1. Control center of medical artificial intelligence with remote system for diagnosis preparation, drug prescription, constituted from a control center (C) in setting up a server hosted on the WEB operating via internet, which includes an information input (II) communicating with dedicated software (S), **characterized by** the same being developed with medical algorithms and filters based on medical artificial intelligence, said control center (C) integrating also a portal with registered physicians (RP) of any regions, states or countries on the planet.

2. Online medical treatment shipping via telemedicine, according to the control center (C) of claim 1, after remote access by the patient (P) and fill out a form about their health, made from the input information (II) sent, the system is **characterized by** instant preparation by dedicated software (S) of clinical diagnosis, drugs prescription and medical treatment proposition to be given simultaneously to the storage of the results in a database (BD) personalized and individual for each patient (P), in the control center (C), which then identifies the physician specialty (P) according to the case, indicating her/him by accessing the portal of registered physicians (RP).

3. Online medical treatment shipping via telemedicine, according to claims 1 and 2, **characterized by** access to the database (BD) remotely by a physician (P), when, having the results of the patient (P) displayed on the screen, validates the attendance for legalized physician monitoring, in a personalized and remotely manner, transmitting data as information, prescriptions, guidance and medical examinations, encrypted by implanted passwords and in progress monitored by said control center (C).

4. Online medical treatment shipping via telemedicine, according to claims 1, 2 and 3, **characterized by** automatic translation by specific software (with a translator program), performed from the sending of information by the patient (P), access by the physician (P) as well as medical treatment and information exchange adjusting both the language used between patient ( P)/physician (P).

5. Online medical treatment shipping via telemedicine, according to claims 1, 2, 3 and 4, **characterized by** immediate access to the database (BD) by the physician (P), from the control center (C), enabling the personalized service in a quick and plural manner, with large numbers of patients (P), whether in private or public network in any part of the planet regardless of the spoken language.
